# EUROPÄISCHE PATENTSCHRIFT

(11) **EP 0 415 287 B1**
(45) Veröffentlichungstag und Bekanntmachung des Hinweises auf die Patenterteilung: **27.10.1993**
(21) Anmeldenummer: 90116260.2
(22) Anmeldetag: 24.08.1990
(51) Int. Cl.: A23L 1/32, A23L 1/015, C07J 9/00

(54) **Verfahren zur Entfernung von Cholesterin bzw. Cholesterinestern aus Eigelb**
Procedure for removing cholesterol and cholesterol esters from egg-yolk
Procédé pour éliminer le cholestérol et les esters de cholestérol du jaune d'oeuf

(30) Priorität: 26.08.1989 DE 3928258
(43) Veröffentlichungstag der Anmeldung: 06.03.1991
(73) Patentinhaber: SKW Trostberg Aktiengesellschaft, 83308 Trostberg (DE)
(72) Erfinder: Cully, Jan, Dr., D-8200 Rosenheim (DE); Vollbrecht, Heinz-Rüdiger, Dr., D-8226 Altenmarkt (DE); Wiesmüller, Johann, D-8261 Engelsberg (DE)
(74) Vertreter: Huber, Bernhard, Dipl.-Chem.

(56) Entgegenhaltungen:
- EP-A- 0 326 469

## Beschreibung

Die Erfindung betrifft ein mehrstufiges Verfahren zur Entfernung von Cholesterin und Cholesterinestern aus Eigelb.

Cholesterin und Cholesterinester sind lipophile Substanzen, die in zahlreichen wichtigen Lebensmitteln tierischen Ursprungs, wie z.B. Eigelb, Fleisch, tierischen Fetten usw. vorkommen.

Erhöhte Cholesterinwerte im Blutserum des Menschen gelten als Risikofaktor für Arteriosklerose bzw. eine koronare Herzkrankheit.

Durch eine Reduzierung der Cholesterinzufuhr durch Lebensmittel wird in pathologischen Fällen angestrebt, die normalen Cholesterinwerte im Blutserum wieder zu erreichen. Aus diesem Grund besteht großes Interesse an einer deutlichen Reduzierung des Cholesterins bzw. der Cholesterinester in fettreichen Lebensmitteln tierischen Ursprungs.

Ein wesentliches Problem ist hierbei, die sensorischen und ernährungsphysiologischen Eigenschaften der Lebensmittel weitgehend zu erhalten.

Es ist zwar schon eine Reihe von Verfahren zur Isolierung von Cholesterin bzw. Cholesterinestern bekannt geworden, doch eignen sich diese Methoden nicht zur Reduzierung des Cholesteringehaltes in Lebensmitteln, da sie chemische Veränderungen wichtiger Bestandteile des Ausgangsmaterials (wie z.B. Proteine, Triglyceride usw.) verursachen.

Ein relativ schonendes Verfahren, welches in jüngster Zeit bekannt geworden ist, bedient sich für die Entfernung des Cholesterins bzw. der Cholesterinester der CO₂-Hochdruckextraktion (vgl. V. Krukonis, Supercritical Fluid Processing, International Symposium on Supercritical Fluids, Nice, 1988).

Dieses Verfahren zeichnet sich zwar durch die physiologische Unbedenklichkeit des Extraktionsmittels (CO₂) aus, doch ist das Arbeiten bei hohem Druck technisch ziemlich aufwendig. Außerdem lassen sich mit diesem Verfahren Cholesterin bzw. Cholesterinester bei schonenden Bedingungen nicht selektiv entfernen, weil auch Triglyceride mitextrahiert werden. Eine Verbesserung der Selektivität durch Temperaturerhöhung ist zwar grundsätzlich möglich, doch wirkt sich diese negativ auf die Beladung des CO₂ mit Cholesterin bzw. Cholesterinestern und auf die Qualität des erhaltenen Produktes aus.

Aus der EP-A1-0 326 469 ist es bekannt, steroidische Verbindungen aus einer biologischen Substanz mit Cyclodextrin in wäßrigem Medium bei einer Temperatur zwischen 20 und 80°C durch Komplexbildung zu adsorbieren und den gebildeten Komplex dann abzutrennen. Als biologische Substanz wird dabei auch Eigelb erwähnt. Dieses Verfahren hat jedoch den Nachteil, daß es relativ hohe Temperaturen und lange Behandlungszeiten benötigt. Dies ist aufgrund der Gefahr bakterieller Kontamination nicht akzeptabel.

Der Erfindung liegt daher die Aufgabe zugrunde, ein Verfahren zur Entfernung des Cholesterins oder/und der Cholesterinester aus Eigelb zu schaffen, welches die genannten Nachteile des Standes der Technik nicht aufweist, sondern mit geringem technischem Aufwand und unter schonenden Bedingungen eine weitgehend selektive Reduzierung dieser Stoffe ermöglicht ohne die Qualität des Eigelbs zu beeinträchtigen.

Diese Aufgabe wird erfindungsgemäß dadurch gelöst, daß man
a) das Eigelbplasma von der LDL-Granulafraktion abtrennt,
b) Cholesterin und Cholesterinester, welche im Eigelbplasma enthalten sind, an ein festes Adsorptionsmittel adsorbiert und
c) den mit Cholesterin oder/und Cholesterinestern beladenen Feststoff von der flüssigen Plasmaphase abtrennt und gegebenenfalls das Eigelbplasma und die abgetrennte Granulafraktion wieder zusammenführt.

Es hat sich nämlich überraschenderweise gezeigt, daß man auf diese Weise Eigelbprodukte mit niedrigem Gesamtcholesteringehalt und guten sensorischen Eigenschaften erhält.

Beim Verfahren der Erfindung wird in der ersten Stufe eine Fraktionierung des Eigelbs in der Weise vorgenommen, daß man das Eigelbplasma von der LDL (= Low Density Lipoprotein)-Granulafraktion abtrennt. Dieser Abtrennungsschritt kann nach bekannten Methoden wie z.B. Filtration und Zentrifugation vorgenommen werden, wobei die Zentrifugation bevorzugt ist, weil sie eine besonders rasche und effektive Trennung ermöglicht. Die Zentrifugation kann bei hierfür üblichen g-Kräften (vorzugsweise bei 1000 bis 10.000 g) durchgeführt werden.

Es empfiehlt sich, zur besseren Abtrennung des Eigelbplasmas (welches ca. 80 bis 85 Gew.-% des Eigelbs ausmacht) von der Granulafraktion, ein emulsionsbrechendes Mittel zuzusetzen, um so die Trennwirkung bzw. Trenngeschwindigkeit zu verbessern. Im Rahmen der Erfindung können im Prinzip alle durch das Lebensmittelgesetz zugelassenen Substanzen eingesetzt werden, die emulsionsbrechende Eigenschaften aufweisen. Als besonders vorteilhaft hat sich hierbei Wasser erwiesen, weil es kostengünstig zur Verfügung steht und anschließend wieder leicht abgetrennt werden kann. Im Falle von Wasser ist es ausreichend, es in einer Menge von 10 bis 200 Gew.-%, insbesondere 50 bis 100 Gew.-%, bezogen auf das Ausgangsgewicht an Eigelb, einzusetzen, um eine relativ leichte Abtrennung zu erreichen.

In der nachfolgenden zweiten Stufe des erfindungsgemäßen Verfahrens erfolgt dann die Entfernung des Cholesterins oder/und der Cholesterinester aus dem Eigelbplasma durch Adsorption an einem geeigneten festen Adsorptionsmittel.

Als Adsorptionmittel können die üblichen, unpolaren Stoffe wie z.B. Aktivkohle, Reverse-Phase-Kieselgel u.ä. verwendet werden.

Als besonders vorteilhaft hat sich der Einsatz von β-Cyclodextrin erwiesen, da es eine besonders selektive Adsorption des Cholesterins bzw. der Cholesterinester in Gegenwart der im Eigelbplasma enthaltenen Begleitsubstanzen ermöglicht.

Die Menge des Adsorptionsmittels kann in weiten Grenzen variiert werden, doch werden vorzugsweise 3 bis 40 Gew.-% Adsorbens, bezogen auf die Trockenmasse des Eigelbplasmas, eingesetzt.

Bei dieser Beladung des Adsorbens, die nach bekannten Methoden wie z.B. durch einfaches Mischen oder Rühren erfolgen kann, werden je nach Art und Menge an eingesetztem Adsorbens etwa 60 bis 99 % des vorhandenen Cholesterins oder/und der Cholesterinester entfernt, während die übrigen Plasmabestandteile weitgehend in der flüssigen Phase verbleiben.

In der dritten Stufe des erfindungsgemäßen Verfahrens wird das mit Cholesterin bzw. Cholesterinestern beladene Adsorbens von der flüssigen Plasmaphase abgetrennt, wobei grundsätzlich auf die in der Technik üblichen physikalischen Verfahren und Methoden zur Trennung von Feststoffen und Flüssigkeiten zurückgegriffen werden kann. Wegen der raschen und vollständigen Trennung wird bevorzugt die Zentrifugation eingesetzt. Natürlich sind aber auch andere Trennverfahren, wie z.B. die Filtration geeignet.

Im allgemeinen kann das Adsorbens nach Desorption der adsorbierten Substanzen wieder verwendet werden.

Die auf diesem Wege von Cholesterin oder/und Cholesterinestern befreite Eigelbplasmafraktion kann dann direkt zu Eigelbprodukten weiterverarbeitet werden. Es empfiehlt sich jedoch aus Gründen der geschmacklichen Qualität, die in der ersten Stufe abgetrennte Granulafraktion, die noch ca. 10 bis 15 % des Gesamtcholesterins enthält, in der cholesterinarmen Plasmafraktion zu redispergieren.

Falls erwünscht und erforderlich, kann noch abschließend das emulsionsbrechende Mittel entfernt werden, welches im Falle von Wasser beispielsweise durch einfache Sprühtrocknung bewerkstelligt werden kann. Auf diese Weise erhält man je nach Trocknungsgrad ein Flüssigei oder ein Eigelbpulver mit einem Gesamtcholesteringehalt von ca. 0,1 bis 0,7 %, was einer ca. 70 bis 90 %igen Reduzierung des Cholesteringehaltes entspricht. Aufgrund dieser guten Reduzierung des Cholesteringehaltes verbunden mit den weiteren Vorteilen wie geringer technischer Aufwand und gute sensorische Qualität der erhaltenen Eigelbprodukte eignet sich das erfindungsgemäße Verfahren besonders gut für die technische Durchführung.

Die nachfolgenden Beispiele sollen die Erfindung näher erläutern

### Beispiel 1

2 kg Eigelb mit einem Gesamtcholesteringehalt von 1,2 % wurden mit destilliertem Wasser in einem Gewichtsverhältnis von 1 : 1 vermischt und 15 Minuten bei 4°C und 6000 g zentrifugiert.

Man erhielt 3,4 kg Plasmafraktion als Überstand. Diese wurde von der Granulafraktion durch Dekantieren getrennt und mit 238 g β-Cyclodextrin 60 Minuten lang durch Rühren innig vermischt. Danach wurde das beladene β-Cyclodextrin durch Zentrifugation aus der flüssigen Plasmaphase abgetrennt.

Als Produkt wurde eine Plasmafraktion mit einem Gesamtcholesteringehalt von 0,01 % erhalten. Diese wurde mit der abgetrennten Granulafraktion wieder zusammengeführt und durch eine Sprühtrocknung zu einem Eigelbpulver mit einem Gesamtcholesteringehalt von 0,2 % verarbeitet, was einer 90 %igen Reduzierung an Cholesterin gegenüber einem unbehandelten Eigelbpulver entspricht.

### Beispiel 2

2 kg Eigelb wurden entsprechend Beispiel 1 in eine Plasmafraktion und eine Granulafraktion durch Zentrifugation aufgetrennt.

3,4 kg der Plasmafraktion wurden anschließend mit 170 g β-Cyclodextrin 60 Minuten lang durch Rühren innig vermischt. Danach wurde das beladene β-Cyclodextrin durch Zentrifugation entsprechend Beispiel 1 von der flüssigen Phase abgetrennt. Auf diese Weise wurde eine Plasmafraktion mit einem Gesamtcholesteringehalt von 0,13 % erhalten. Diese wurde mit der abgetrennten Granulafraktion zusammengeführt und durch Sprühtrocknung zu einem Eigelbpulver mit einem Gesamtcholesteringehalt von 0,62 % verarbeitet, was einer 72 %igen Reduzierung an Cholesterin gegenüber einem unbehandelten Eigelbpulver entspricht.

## Patentansprüche

1. Verfahren zur Entfernung von Cholesterin bzw. Cholesterinestern aus Eigelb durch Adsorption an ein festes Adsorptionsmittel und Entfernen des gebildeten Adsorbates,
**dadurch gekennzeichnet**,
daß man
(a) das Eigelbplasma von der LDL-Granulafraktion abtrennt,
(b) Cholesterin und Cholesterinester, welche im Eigelbplasma enthalten sind, an ein festes Adsorptionsmittel adsorbiert und
(c) den mit Cholesterin oder/und Cholesterinestern beladenen Feststoff von der flüssigen Plasmaphase abtrennt und gegebenenfalls das Eigelbplasma und die abgetrennte Granulafraktion wieder zusammenführt.

2. Verfahren nach Anspruch 1,
**dadurch gekennzeichnet**,
daß man dem Eigelb vor Abtrennung der LDL-Granulafraktion ein emulsionsbrechendes Mittel zusetzt.

3. Verfahren nach Anspruch 1,
**dadurch gekennzeichnet,**
daß man die Abtrennung des Eigelbplasmas von der Granulafraktion durch Zentrifugation vornimmt.

4. Verfahren nach Anspruch 3
**dadurch gekennzeichnet,**
daß man als emulsionsbrechendes Mittel Wasser einsetzt.

5. Verfahren nach Anspruch 4,
**dadurch gekennzeichnet,**
daß man Wasser in einer Menge von 10 bis 200 Gew.-%, bezogen auf das Ausgangsgewicht des Eigelbes, einsetzt.

6. Verfahren nach Anspruch 5,
**dadurch gekennzeichnet,**
daß man Wasser in einer Menge von 50 bis 100 Gew.-% verwendet.

7. Verfahren nach einem der Ansprüche 1 bis 6,
**dadurch gekennzeichnet,**
daß man als festes Adsorptionsmittel für die Adsorption des Cholesterins oder/und Cholesterinester β-Cyclodextrin verwendet.

8. Verfahren nach einem der Ansprüche 1 bis 7,
**dadurch gekennzeichnet,**
daß man das Adsorptionsmittel in einer Menge von 3 bis 40 Gew.-%, bezogen auf die Trockenmasse des Eigelbplasmas einsetzt.

9. Verfahren nach einem der Ansprüche 1 bis 8,
**dadurch gekennzeichnet,**
daß man das mit Cholesterin oder/und Cholesterinestern beladene Adsorptionsmittel von der flüssigen Plasmaphase abzentrifugiert.

## Claims

1. Process for the removal of cholesterol and cholesterol esters from egg yolk by adsorption on a solid adsorption agent and removal of the adsorbate formed, characterised in that one
a) separates the egg yolk plasma from the LDL-granula fraction,
b) adsorbs cholesterol and cholesterol esters which are contained in the egg yolk plasma on a solid adsorption agent and
c) separates off the solid material loaded with cholesterol and/or cholesterol esters from the liquid plasma phase and possibly again mixes together the egg yolk plasma and the separated granula fraction.

2. Process according to claim 1, characterised in that one carries out the separation of the egg yolk plasma from the granula fraction by centrifuging.

3. Process according to claim 1, characterised in that, before separation of the LDL granula fraction, one adds an emulsion-breaking agent to the egg yolk.

4. Process according to claim 3, characterised in that one adds water as emulsion-breaking agent.

5. Process according to claim 4, characterised in that one uses water in an amount of 10 to 200 wt.%, referred to the starting weight of the egg yolk.

6. Process according to claim 5, characterised in that one uses water in an amount of from 50 to 100 wt.%.

7. Process according to one of claims 1 to 6, characterised in that, as solid adsorption agent for the adsorption of the cholesterol and/or cholesterol esters, one uses β-cyclodextrin.

8. Process according to one of claims 1 to 7, characterised in that one uses the adsorption agent in an amount of from 3 to 40 wt.%, referred to the dry weight of the egg yolk plasma.

9. Process according to one of claims 1 to 8, characterised in that one centrifuges off the adsorption agent loaded with cholesterol and/or cholesterol esters from the liquid plasma phase.

## Revendications

1. Procédé pour éliminer le cholestérol ou les esters de cholestérol du jaune d'oeuf par adsorption sur un agent d'adsorption solide et élimination de l'adsorbat formé,
caractérisé en ce que
a) l'on sépare le plasma du jaune d'oeuf de la fraction granulaire LDL,
b) on adsorbe le cholestérol et les esters de cholestérol qui sont contenus dans le plasma du jaune d'oeuf sur un agent d'adsorption solide, et
c) on sépare la matière solide chargée de cholestérol ou/et d'esters de cholestérol de la phase de plasma liquide et éventuellement on réunit à nouveau le plasma de jaune d'oeuf et la fraction granulaire séparée.

2. Procédé selon la revendication 1, caractérisé en ce que l'on procède à la séparation du plasma de jaune d'oeuf de la fraction granulaire par centrifugation.

3. Procédé selon la revendication 1, caractérisé en ce que l'on ajoute au jaune d'oeuf, avant séparation de la fraction granulaire LDL, un agent antiémulsion.

4. Procédé selon la revendication 3, caractérisé en ce que l'on utilise de l'eau en tant qu'agent antiémulsion.

5. Procédé selon la revendication 4, caractérisé en ce que l'on utilise de l'eau dans une quantité de 10 à 200% en poids rapportés au poids de départ du jaune d'oeuf.

6. Procédé selon la revendication 5, caractérisé en ce que l'on utilise de l'eau dans une quantité de 50 à 100% en poids.

7. Procédé selon les revendications 1 à 6, caractérisé en ce que comme agent d'adsorption solide, on utilise pour l'adsorption du cholestérol et/ou des esters de cholestérol de la β-cyclodextrine.

8. Procédé selon l'une quelconque des revendications 1 à 7, caractérisé en ce que l'agent d'adsorption est mis en oeuvre dans une quantité de 3 à 40 % en poids rapportés à la masse sèche du plasma de jaune d'oeuf.

9. Procédé selon l'une quelconque des revendications 1 à 8, caractérisé en ce que l'on sépare par centrifugation l'agent d'adsorption chargé de cholestérol ou/et d'esters de cholestérol de la phase de plasma liquide.
